# EUROPEAN PATENT SPECIFICATION

(11) **EP 4 504 071 B1**
(45) Date of publication and mention of the grant of the patent: **09.12.2026**
(21) Application number: 22721666.0
(22) Date of filing: 07.04.2022
(51) Int. Cl.: A61B 17/00, A61B 17/12, A46B 3/00, A61B 17/32, A46B 3/08, A46B 3/18, A46B 3/20, A46D 3/04, A46D 3/05

(54) **VENOUS ABLATION DEVICE**
VENENABLATIONSVORRICHTUNG
DISPOSITIF D'ABLATION VEINEUSE

(43) Date of publication of application: 12.02.2025
(73) Proprietor: Clearstream Technologies Limited, Enniscorthy, County Wexford (IE)
(72) Inventor: WALL, Sean, Enniscorthy, County Wexford (IE)
(74) Representative: Hoffmann Eitle
(86) International application number: PCT/EP2022/059229
(87) International publication number: WO 2023/193910

(56) References cited:
- WO-A1-2021/156343
- US-A1- 2002 010 418
- US-A1- 2007 248 640
- US-A1- 2007 282 359
- US-A1- 2014 276 388
- US-A1- 2019 125 322
- US-A1- 2021 244 417

## Description

### TECHNICAL FIELD

The present disclosure relates to venous ablation devices. More particularly the present disclosure relates to a non-thermal, non-tumescent venous ablation device.

### BACKGROUND

Superficial venous reflux and chronic venous insufficiency, presenting as varicose veins in the lower limbs, may be treated by a number of different methods. Some treatment methods involve thermal ablation of the affected vessel, for example by radiofrequency ablation or endovenous laser ablation, which causes significant patient pain. Some methods include the use of tumescent fluids during treatment.

US 2007/282359 A1 discloses an ablation apparatus formed with a brush at its distal end, wherein the apparatus includes a conduit for the application of sclerosing agent to the treatment site and is further provided with a sheath facilitating position of the apparatus at a desired treatment site.

Non-thermal, non-tumescent treatments involve the use of implanted materials like sclerosants and cyanoacrylate adhesives. There is a need for non-thermal, non-tumescent treatments which do not use such implanted materials.

### SUMMARY

The invention is defined by claims 1 and 10. further embodiments of the invention are defined by the dependent claims. Methods for surgery are not claimed.

According to a first aspect there is provided a venous ablation device for denuding a vessel wall, comprising: a bristle portion, the bristle portion comprising a core and a plurality of resiliently flexible bristles extending at least radially outwardly from the core, the bristles having a collapsed delivery configuration and an expanded deployed configuration in which the bristles are configured to contact and denude the vessel wall; and a delivery portion configured to deliver the bristle portion through a delivery catheter, the delivery portion at least partially integral with the core; wherein the plurality of bristles penetrate through the core such that the core secures the bristles.

According to a second aspect there is provided a method of manufacturing a venous ablation device, comprising: providing a core and a delivery portion configured to deliver the bristle portion through a delivery catheter, the delivery portion at least partly integral with the bristle portion; providing a plurality of resiliently flexible bristles to the core so that the plurality of bristles penetrate through the core; and securing the bristles to the core so that the plurality of bristles extend at least radially outwardly from the core the bristles having a collapsed delivery configuration and an expanded deployed configuration in which the bristles are configured to contact and denude the vessel wall.

According to a third aspect there is provided a method of closing a blood vessel of a patient, comprising the steps of: inserting a delivery catheter into the vasculature of the patient; positioning a distal portion of the delivery catheter at a target site in the blood vessel; delivering a venous ablation device according to the first aspect to the distal portion of the delivery catheter; deploying the venous ablation device from the delivery catheter so that the flexible bristles contact the vessel wall; and translating the venous ablation device to denude the vessel wall.

### BRIEF DESCRIPTION OF THE DRAWINGS

To enable better understanding of the present disclosure, and to show how the same may be carried into effect, reference will now be made, by way of example only, to the accompanying schematic drawings, in which:
Fig. 1A shows a schematic side view of a venous ablation device in a first configuration according to one or more embodiments shown and described herein;
Fig. 1B shows a schematic side view of the venous ablation device of Fig. 1A in a second configuration according to one or more embodiments shown and described herein;
Fig. 1C shows a schematic side view of a flexible bristle for a venous ablation device according to one or more embodiments shown and described herein;
Fig. 2 shows a schematic side view of a venous ablation device according to one or more embodiments shown and described herein;
Fig. 3A shows a schematic perspective view of a method of manufacturing a venous ablation device according to one or more embodiments disclosed herein;
Fig. 3B shows a schematic perspective view of a venous ablation device manufactured according to the method of Fig. 3A according to one or more embodiments disclosed herein;
Fig. 4 shows a schematic cross-sectional side view of a venous ablation device according to one or more embodiments disclosed herein;
Fig. 5 shows a schematic cross-sectional side view of another venous ablation device according to one or more embodiments disclosed herein;
Fig. 6 shows a schematic cross-sectional side view of another venous ablation device according to one or more embodiments disclosed herein;
Fig. 7 shows a schematic cross-sectional side view of another venous ablation device according to one or more embodiments disclosed herein; and
Fig. 8 shows a schematic cross-sectional side view of another venous ablation device according to one or more embodiments disclosed herein.

### DETAILED DESCRIPTION

As used herein, the term "venous ablation device" or "NTNT device", may be understood to mean a device which causes mechanical damage to the vessel walls to induce shutdown of the vessel without the use of thermal ablation or of tumescent fluids.

As used herein, the term "integral", in the context of one element being integral with another element, may be understood to mean that the elements form a singular unitary body.

As used herein, a 'collapsed delivery configuration' of an element may refer to a configuration of the element which has a smaller radial extent than an 'expanded deployed configuration' of the element.

Throughout this disclosure, the term 'tube wall' may refer to the wall of a tube which extends along the longitudinal axis of the tube. The tube wall may be continuous or discontinuous. The tube may take any cross-sectional shape, for example circular, oval or square. The tube may take one or multiple different cross-sectional shapes along its length.

Throughout this disclosure, the term 'tube' may refer to any element which has a tube wall in which different portions of the tube wall oppose each other across a longitudinal axis. For example, the tube wall may be curved (e.g. having a circular cross-section) around the longitudinal axis of the tube such that opposite sides of the tube wall oppose each other. The tube may or may not have a lumen extending along any portion of its longitudinal axis.

Throughout this disclosure, an element referred to as being 'clamped' by another clamping element, refers to the clamping element directly or indirectly providing forces (for example, opposing forces) on the element so as to restrain the element. The clamping element may directly or indirectly contact the clamped element.

Throughout this disclosure, a 'shrinkable material' may refer to a material which shrinks in a particular direction upon a particular treatment. Such a treatment may be a heat and/or or chemical treatment. As would be understood by the skilled person, the shrinkable materials themselves shrink without an external force being applied to them, for example, by crimping.

Throughout this disclosure reference is made to a delivery portion being integral with a core of a bristle portion. Where the delivery portion and/or bristle portion comprises a plurality of elements, it will be understood to mean that at least one element of the delivery portion is integral with at least one element of the bristle portion. In other words, the delivery portion may be at least partly integral with the bristle portion. As such, the devices disclosed herein are not configured to be implanted into the body and under normal use of the device the bristle portion remains integral with the delivery portion.

Throughout this disclosure reference is made to NTNT devices comprising a plurality of flexible bristles. It will be understood that the term "flexible bristle" refers to any bristle which is resiliently deformable. In any of the devices disclosed herein, the flexible bristles may be made of any resiliently deformable material such as stainless steel, Elgiloy or Nitinol. Other suitable materials may also be used, such as any suitable polymer or composite or any other shape memory metal or metal alloy. The diameter of an individual flexible bristle may range from 0.036mm (0.0014 inches) to 0.053mm (0.0021 inches). For example, the diameter of an individual flexible bristle may be 0.0381mm (0.0015 inches), 0.0445mm (0.00175 inches) or 0.0508mm (0.002 inches). The diameter of an individual flexible bristle may be greater than 0.1mm, and in some exmaples up to 0.15mm. The average radial diameter of the radial profile of the NTNT device formed by the expanded flexible bristles in an unconstrained configuration may range from 5mm to 30mm.

It will further be understood that the flexible bristles are of a sufficient stiffness to be able to cause damage to the vessel wall. In some embodiments, the stiffness of the flexible bristles (i.e. modulus of elasticity) may be at least 3 GPa, for example between 3 GPa and 310 GPa.

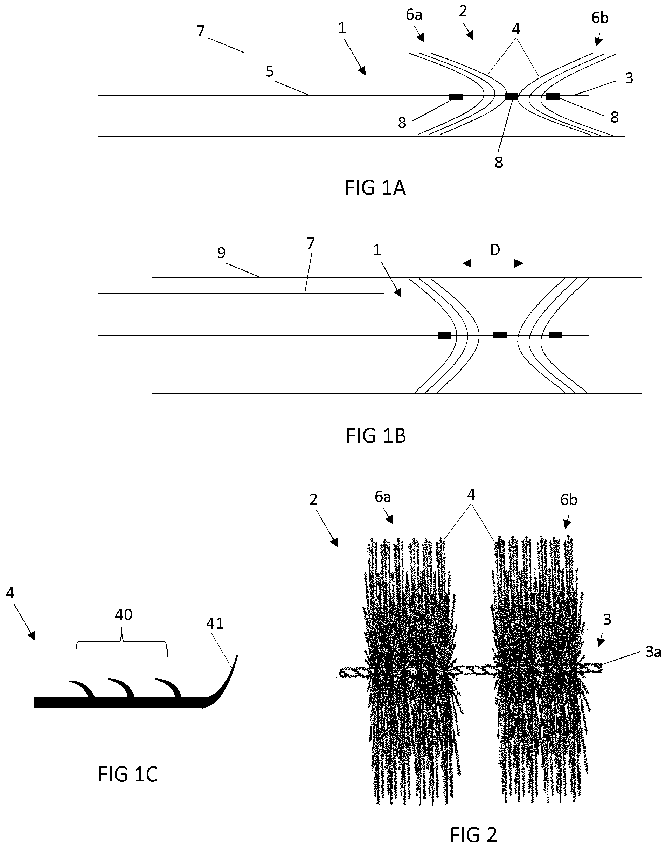

Fig. 1A shows a schematic side view of a non-thermal, non-tumescent venous ablation device 1 in a delivery configuration. The venous ablation device 1 is suitable for denuding a vessel wall. The venous ablation device 1 comprises a bristle portion 2. The bristle portion 2 comprises a core 3 and a plurality of resiliently flexible bristles 4 extending at least radially outwardly from the core 3, and partially longitudinally along the core 3. The bristles 4 have a collapsed delivery configuration, as shown in Fig. 1A, in which the bristles 4 fit inside a delivery catheter 7 for delivering the venous ablation device 1 to a target site in the vasculature. The bristles 4 also have an expanded delivery configuration, as shown in Fig. 1B, in which the bristles are configured to contact and denude the vessel wall 9. The venous ablation device 1 also comprises a delivery portion 5 configured to deliver the bristle portion 2 through the delivery catheter 7. The delivery portion 5 may be any suitable element configured to slidably fit inside the delivery catheter 7 and having a length such that it is extendable from a proximal portion of the delivery catheter 7 to a distal portion of the delivery catheter 7. The delivery portion 5 is at least partially integral with the core 3, that is to say, at least one element of the delivery portion 5 forms a unitary body with at least one element of the bristle portion 2, and in particular at least one element of the core 3. The plurality of bristles 4 each penetrate through the core 3 such that the core 3 secures the bristles 4 to the bristle portion 2. The venous ablation device 1 may comprise one or more radiopaque markers 8 which are viewable under radiography for locating the venous ablation device 1. The radiopaque markers 8 may be provided at any suitable location on the venous ablation device 1, for example proximal to the most proximal bristle 4 of the device 1, longitudinally between the ends of neighbouring bristle segments, within a bristle segment or distal to the most distal bristle 4 of the device 1. For example, the radiopaque markers 8 may comprise an annulus or tube or radiopaque material mounted on the exterior of the core 3.

Fig. 1B shows the venous ablation device 1 of Fig. 1A in a second, expanded deployed configuration. In use, the delivery catheter 7 is navigated to a target site in the vasculature, for example a varicose vein or other vessel for ablation. The venous ablation device 1 is loaded into the delivery catheter 7 from a proximal end of the delivery catheter 7, and the delivery portion 5 is pushed in a distal direction through the delivery catheter 7 so that the bristle portion 2 reaches the distal tip of the delivery catheter 7. To deploy the venous ablation device 1, the delivery catheter 7 is moved proximally relative to the venous ablation device 1 (or the venous ablation device 1 is moved distally relative to the delivery catheter 7) such that the venous ablation device 1 is exposed from the distal tip of the delivery catheter 7, as shown in Fig. 1B. The resiliently flexible bristles 4 self-expand from the collapsed delivery configuration to the expanded deployed configuration in which the bristles 4 contact the vessel wall 9. The venous ablation device 1 is then moved proximally and distally and/or rotated such that the bristles 4 denude or score the venous endothelium, which elicits a local reaction that results in shutting down the vessel and complete closure of the varicose veins. Advantageously, diseased or varicosed veins with stagnant or refluxing flow rapidly shut down when treated by denudation or scoring, whereas healthy veins with normal blood flow do not respond as readily to this treatment.

In some embodiments and in accordance with the present claimed invention, as illustrated in Fig. 1A, the venous ablation device 1 comprises a first set 6a of bristles 4 and a second set 6b of bristles 4, wherein the first set 6a of bristles 4 are configured to extend in a proximal direction in the expanded deployed configuration (as shown in Fig. 1B), and the second set 6b of bristles 4 are configured to extend in a distal direction in the expanded deployed configuration (as shown in Fig. 1B), the distal direction opposite to the proximal direction. In some embodiments, not in accordance with the present claimed invention, the bristles 4 extend in a single longitudinal direction in the deployed configuration (either distally or proximally, but not both). That is, in some embodiments, the venous ablation device 1 may comprise only bristles 4 extending distally, or only bristles 4 extending proximally. The first and second sets 6a, 6b of bristles 4 may each be grouped into a single segment located away from the other sets of bristles 4, as illustrated in Fig. 1A (i.e. there may be provided a gap between the bristle segments which is larger than the gap between neighbouring bristles within a single segment). The venous ablation device 1 may comprise more than two bristle segments, wherein each segment comprises distally or proximally extending bristles 4. Having a set of bristles 4 that point distally and a set of bristles that point proximally in the venous ablation device 1 may cause damage to the vessel wall 9 when the venous ablation device 1 is moved proximally and distally, rather than only causing damage in a single direction of movement.

Fig. 1C shows a schematic view of a flexible bristle 4 according to one or more embodiments. The flexible bristle 4 may comprise one or more hooks or barbs 40 along the length of the bristle 4. Alternatively or additionally the flexible bristle 4 may comprise one or more hooks or barbs 41 at the tip of the bristle 4. It is noted that in any of the embodiments disclosed herein, one or more of the flexible bristles of the venous ablation device may be provided with such one or more hooks or barbs 40, 41 disposed along the length of the bristles 4 and/or at the tips of the bristles 4. The hooks or barbs 40, 41 may be attached to the flexible bristles 4 by adhesive or welding. The hook or barb 41 at the tip of the bristle 4 may be unitary with the bristle 4 and the bristle 4 may be a shape memory material (such as nitinol) which is shape-set to include a hook or barb 41 shape at its tip. Accordingly, when deployed, the flexible bristle 4 expands to its pre-deformed shape including the hook or barb 41. The provision of one or more hooks or barbs 40, 41 may increase the damage provided to the vessel wall by the venous ablation device.

Fig. 2 shows a schematic view of a bristle portion 2 of an NTNT venous ablation device 1 in an unconstrained configuration according to one or more embodiments. For example, the bristle portion 2 shown in Fig. 2 may be provided in the venous ablation device 1 described with reference to Figs. 1A and 1B. In the embodiment shown in Fig. 2, the bristle portion 2 comprises a core 3 comprising a twisted wire portion 3a, wherein the plurality of bristles 4 penetrate the core 3 and are clamped by the twisted wire portion 3a such that the bristles 4 are secured to the core 3. The bristles 4 may be additionally secured to the core 3 by, for example, adhesive or welding. The bristle portion 2 may comprise a first set of bristles 6a configured to extend proximally and a second set of bristles 6b configured to extend distally, the distal direction opposite to the proximal direction. In some embodiments, the bristles 4 extend in a single longitudinal direction in the deployed configuration (either distally or proximally, but not both). That is, in some embodiments, the bristle portion 2 of Fig. 2 may comprise only bristles 4 extending distally, or only bristles 4 extending proximally. The first and second sets 6a, 6b of bristles 4 may each be grouped into a single segment located away from the other sets of bristles, as illustrated in Fig. 2 (i.e. there may be provided a gap between the bristle segments which is larger than the gap between neighbouring bristles within a single segment). The venous ablation device 1 may comprise more than two bristle segments, wherein each segment comprises distally or proximally extending bristles 4. The twisted wire portion 3a may be made of flexible twisted metal wire, for example, stainless steel or any other suitable material. In some embodiments, the diameter of the twisted wire portion 3a used may be 0.5mm or less, though other diameters are contemplated and possible. The twisted wire portion 3a is at least partially integral with the delivery portion 5 (Fig. 1A) of the venous ablation device 1. For example, the delivery portion 5 may comprise one or more of the wires forming the twisted wire portion 3a configured to extend through the delivery catheter 7 (Fig. 1A) from a proximal end to a distal end of the delivery catheter 7.

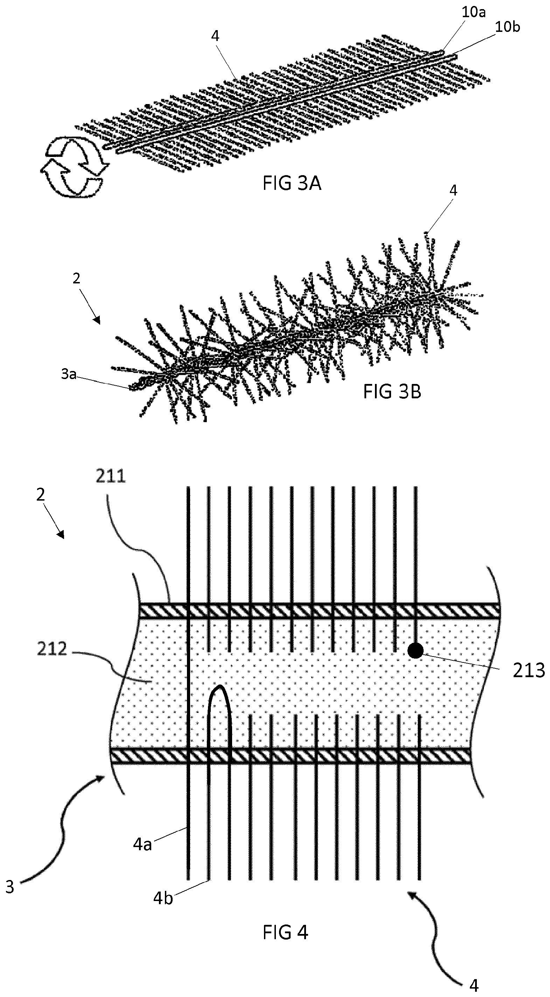

Figs. 3A and 3B show a method of manufacturing the bristle portion 2 described with reference to Fig. 2, using a twisted wire method. A plurality of flexible bristles 4 may be place between two parallel wires 10a, 10b. The parallel wires 10a, 10b may be fixed at one end and twisted about each other at another end to form a twisted wire portion. Upon twisting, the wires 10a, 10b are formed into a helix causing the bristles 4 to translate from being parallel to being rotationally offset from one another about the twisted wire portion 3a, wherein the twisted wire portion 3a clamps the bristles 4. Variations in the bristle density can be achieved by varying the pitch of the twisted wire which clamps the bristles 4. Fig. 3B shows the bristle portion 2 formed after the wires 10a and 10b are twisted about each other. In the embodiment of Figs. 3A and 3B, the twisted wire portion 3a therefore comprises two wire portions 10a and 10b twisted about each other. The delivery portion 5 (Fig. 1A) may comprise one or both of the wire portions 10a, 10b extendable from a proximal portion to a distal portion of a delivery catheter for delivering the bristle portion 2 to the bodily lumen. In the delivery portion 5, the wire portions 10a and 10b may also be twisted about one another, or they may extend in an untwisted manner (as the delivery portion 5 does not need to be twisted to clamp any bristles 4). It will be noted that the bristle portion 2 shown in Fig. 2 may be manufactured by other methods, for example by first forming a twisted wired portion 3a and second mounting and securing the bristles 4 to the twisted wire portion 3a.

Fig. 4 shows a cross-sectional schematic view of a bristle portion 2 of an NTNT venous ablation device 1 in an unconstrained configuration according to one or more embodiments. For example, the bristle portion 2 shown in Fig. 4 may be provided in the venous ablation device 1 described with reference to Figs. 1A and 1B. In the embodiment shown in Fig. 4, the core 3 of the bristle portion 2 comprises a tube having a tubular wall 211. The plurality of bristles 4 each penetrate the tube wall 211 so that the tube wall 211 secures the plurality of bristles 4 to the core 3. The plurality of bristles 4 extend at least radially outwardly from the core 3. The tube wall 211 may comprise a plurality of holes in the wall, with one or more of the plurality of bristles 4 penetrating each hole in the tube wall 211. The tube may be generally cylindrical. The tube may define a lumen along the longitudinal axis of the tube. The bristle portion 2 of Fig. 4 may comprise a first set of bristles 4 configured to extend proximally and a second set of bristles 4 configured to extend distally, the distal direction opposite to the proximal direction. in some embodiments, the bristles 4 extend in a single longitudinal direction in the deployed configuration (either distally or proximally, but not both). That is, in some embodiments, the bristle portion 2 of Fig. 4 may comprise only bristles 4 extending distally, or only bristles 4 extending proximally. The first and second sets of bristles 4 may each be grouped into a single segment located away from the other sets of bristles 4, as illustrated in Fig. 1A (i.e. there may be provided a gap between the bristle segments which is larger than the gap between neighbouring bristles within a single segment). With additional reference to Figs. 1A and 1B, the venous ablation device 1 may comprise more than two bristle segments, wherein each segment comprises distally or proximally extending bristles 4. The core 3 is at least partially integral with the delivery portion 5 of the venous ablation device 1. For example, the delivery portion 5 may comprise one or more of the tube wall 211 and the filler material which are configured to extend from a proximal end of a delivery catheter to a distal end of the delivery catheter.

As shown in Fig. 4, the plurality of flexible bristles 4 each penetrate through the tube wall 211 such that a portion of each of the flexible bristles 4 is disposed within the lumen of the tube.

The tube wall 211 may have a plurality of holes therein (not shown) through which, for example, a single one or some of the plurality of flexible bristles 4 passes therethrough. The holes may each have substantially the same diameter as the flexible bristle 4 which passes therethrough.

In some embodiments, the tube wall 211 may define a hollow lumen. In other embodiments, the core 3 may further comprise filler material 212 disposed within the lumen of the tube. As shown in Figure 4, the filler material 212 may substantially fill the lumen of the tube. The filler material 212 may comprise or consist of: medical grade 2 part epoxy resin, polyurethane, nylon 12, Pebax 4033, liquid crystal polymer, polyether ether ketone, polycarbonate, neoprene, acrylate polymers or any combination thereof.

The filler material 212 disposed within the lumen of the tube may engage the portions of the flexible bristles 4 which are disposed within the lumen of the tube. The filler material 212 may act to additionally secure the plurality of flexible bristles 4 to the core 3.

The filler material 212 may be an adhesive. In such embodiments, the filler material 212 may engage the flexible bristles 4 by adhering or bonding to the flexible bristles 4.

The filler material 212 may be a curable material or settable material, which is curable or settable upon heating, solvent flashing and/or irradiating. Upon curing or setting, the filler material 212 may harden so as to secure the flexible bristles 4 to the core 3.

The filler material 212 may engage the flexible bristles 4 by mechanically anchoring the flexible bristles 4 to the core 3.

As one (or a sub-set) of the plurality of flexible bristles 4 passes through each hole in the tube wall 211, the flexible bristles 4 are attached to the core 3 individually or in a (small) sub-set. Accordingly, if the integrity of the attachment of one of the flexible bristles 4 becomes compromised, the integrity of the attachments of the other flexible bristles 4 may not be compromised. For example, if one of the plurality of flexible bristles 4 is dislodged from the core 3, the attachment of the remaining flexible bristles 4 may remain uncompromised, which is not the case when the majority of the attachment force for a particular bristle 4 is provided for by surrounding/neighbouring bristles 4 (for example in the embodiment shown in Fig. 2, where one bristle 4 becoming dislodged from the twisted wire 3a may reduce the clamping force on neighbouring bristles 4 by the twisted wire 3a, increasing the likelihood that a plurality of bristles 4 become dislodged).

The core 3 and flexible bristles 4 of the venous ablation device may be manufactured by creating, for example, by machining, a plurality of holes in the tube wall 211 of the tube. One of the plurality of flexible bristles 4 may be inserted through each of the plurality of holes in the tube wall 211 such that a portion of each of the flexible bristles 4 extends into the lumen of the tube and a free portion of each of the flexible bristles 4 extends radially outwardly from the tube wall 211.

The filler material 212 may be disposed in the lumen of the tube. The filler material 212 may be disposed in the lumen of the tube before or after inserting the flexible bristles 4.

In certain embodiments, the filler material 212 may be a curable or settable material such that after disposing the filler material 212 in the lumen of the tube, the filler material 212 is cured or set such that it hardens so as to secure the plurality of flexible bristles 4 to the core 3.

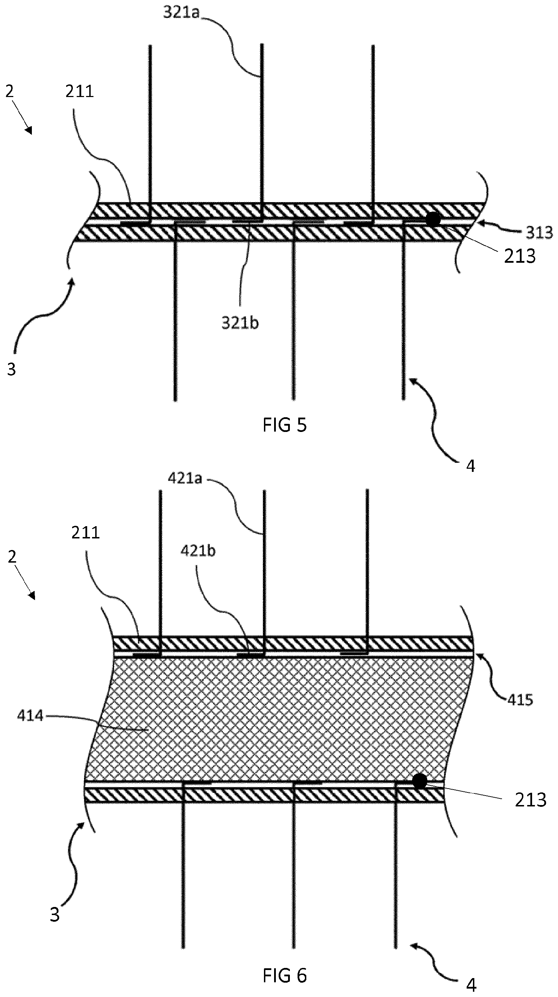

It is noted that the filler material 212 described with reference to Fig. 4 may be provided to any of the bristle portions 2 disclosed herein comprising a tube wall (for example the embodiments described in relation to Figs. 5 and 6).

The plurality of holes in the tube wall 211 may be arranged so as to arrange the flexible bristles 4 in a prescribed manner. For example, the plurality of holes may be oriented such that the flexible bristles 4 are distributed substantially evenly around the circumference of the core 3. Additionally or alternatively, the plurality of holes may be arranged in spaced-apart segments as disclosed above.

Optionally, one or more of the plurality of bristles 4 may comprise a thick portion 213 disposed in the lumen defined by the tubular wall 211, the thick portion 213 being thicker than the portion of the bristle 4 extending through the tubular wall 211. The thick portion 213 may further reduce the likelihood of a bristle 4 becoming dislodged from the tubular wall 211. A thick portion 213 may be provided on any of the bristles 4 in any of the embodiments disclosed herein (for example on any of the bristles 4 of the bristle portions 2 described with reference to Figs. 2 to 8).

The tube wall 211 described in relation to Figs. 4 to 6 may be made of any suitable material, such as a polymer, and in some embodiments may comprise or consist of a heat shrinkable material such as polyolefin, Pebax, FEP, PTFE, PFA, ETFE, PET, polyether ether ketone or any combination thereof.

Fig. 5 shows a schematic cross-sectional view of a bristle portion 2 of an NTNT venous ablation device 1 in an unconstrained configuration according to one or more embodiments. For example, the bristle portion 2 shown in Fig. 5 may be provided in the venous ablation device 1 described with reference to Figs. 1A and 1B. In the embodiment shown in Fig. 5, the core 3 of the bristle portion 2 comprises a tube having a tubular wall 211. The plurality of bristles 4 each penetrate the tube wall 211 so that the tube wall 211 secures the plurality of bristles 4 to the core 3. The plurality of bristles 4 extend at least radially outwardly from the core 3. The tube wall 211 may comprise a plurality of holes in the wall, with one or more of the plurality of bristles 4 penetrating each hole in the tube wall 211. The tube may be generally cylindrical. The tube may define a lumen along the longitudinal axis of the tube. The bristle portion 2 of Fig. 5 may comprise a first set of bristles 4 configured to extend proximally and a second set of bristles 4 configured to extend distally, the distal direction opposite to the proximal direction. In some embodiments, the bristles 4 extend in a single longitudinal direction in the deployed configuration (either distally or proximally, but not both). That is, in some embodiments, the bristle portion 2 of Fig. 5 may comprise only bristles 4 extending distally, or only bristles 4 extending proximally. The first and second sets of bristles 4 may each be grouped into a single segment located away from the other sets of bristles 4, as illustrated in Fig. 1A (i.e. there may be provided a gap between the bristle segments which is larger than the gap between neighbouring bristles within a single segment). With additional reference to Figs. 1A and 1B, the venous ablation device 1 may comprise more than two bristle segments, wherein each segment comprises distally or proximally extending bristles 4. The core 3 is at least partially integral with the delivery portion 5 of the venous ablation device 1. For example, the delivery portion 5 may comprise one or more of the tube wall 211 and the filler material which are configured to extend from a proximal end of a delivery catheter to a distal end of the delivery catheter.

As shown in Fig. 5, the plurality of flexible bristles 4 each penetrate through the tube wall 211 such that a portion of each of the flexible bristles 4 is disposed within the lumen of the tube.

The tube wall 211 may have a plurality of holes therein (not shown) through which, for example, a single one or some of the plurality of flexible bristles 4 passes therethrough. The holes may each have substantially the same diameter as the flexible bristle 4 which passes therethrough.

In some embodiments, the tube wall 211 may define a hollow lumen. In other embodiments, the core 3 may further comprise filler material 212 disposed within the lumen of the tube, as described previously with reference to Fig. 4.

As can be seen from Figure 5, each of the plurality of flexible bristles 4 penetrate through the tube wall 211. A free portion 321a of each of the plurality of flexible bristles 4 extends radially outwardly from the tube, and, in particular, the tube wall 211.

A clamped portion 321b of each of the plurality of flexible bristles 4 is clamped between two opposing sides of the tube wall 211. The opposing sides of the tube wall 211 provide a clamping force on each of the clamped portions 321b such that the flexible bristles 4 are secured to the core 3.

In the embodiment shown in Figure 5, the tube of the core 3 has a lumen 313 extending along substantially the entire length of the tube. In this embodiment, the lumen 313 is a continuous lumen in which the clamped portions 321b of the flexible bristles 4 are disposed within the lumen 313. The opposing sides of the tube wall 211 may not contact each other at regions between the clamped portions 321b such that a continuous lumen is defined.

One portion of the lumen 313 may not be in fluid communication with another portion of the lumen 313, for example, due to a clamped portion 321b entirely filling a section of the lumen 313.

In certain embodiments, the portions of the tube wall 211 between some or all of the clamped portions 321b may be configured such that the opposing sides of the tube wall 211 contact each other (not shown in Figure 5).

Optionally, the portions of the tube wall 211 of the tube between each of the clamped portions 321b may be configured such that the opposing sides of the tube wall 311 contact each other such that the tube may comprise a number of distinct, isolated lumens disposed along the length of the tube. The lumens of the tube may be substantially filled by the clamped portions 321b of the plurality of flexible bristles 4.

The core 3 and flexible bristles 4 of the bristle portion 2 shown in Fig. 5 may be manufactured by creating, for example, by machining, a plurality of holes in the tube wall 211 of the tube. One or some of the plurality of flexible bristles 4 may be inserted through each of the plurality of holes in the tube wall 211 such that a portion 321b of each of the flexible bristles 4 extends into the lumen 313 of the tube.

The portions 321b of each of the flexible bristles 4 may be clamped between two opposing sides of the tube wall 211.

In this regard, at least portions of the tube may be shrunk, for example, heat shrunk or chemically shrunk, in a radial direction such that the portions 321b of each of the flexible bristles 4 are clamped between two opposing sides of the tube wall 211.

Additionally or alternatively, at least portions of the tube may be compressed (e.g. mechanically compressed), for example, by crimping, in a radial direction such that the portions 321b of each of the flexible bristles 4 are clamped between two opposing sides of the tube wall 211.

One or more of the bristles of Fig. 5 may comprise a thick portion 213 as previously disclosed with reference to Fig. 4.

Fig. 6 shows a schematic cross-sectional view of a bristle portion 2 of an NTNT venous ablation device 1 in an unconstrained configuration according to one or more embodiments. For example, the bristle portion 2 shown in Fig. 6 may be provided in the venous ablation device 1 described with reference to Figs. 1A and 1B. In the embodiment shown in Fig. 6, the core 3 of the bristle portion 2 comprises a tube having a tubular wall 211. The plurality of bristles 4 each penetrate the tube wall 211 so that the tube wall 211 secures the plurality of bristles 4 to the core 3. The plurality of bristles 4 extend at least radially outwardly from the core 3. The tube wall 211 may comprise a plurality of holes in the wall, with one or more of the plurality of bristles 4 penetrating each hole in the tube wall 211. The tube may be generally cylindrical. The tube may define a lumen along the longitudinal axis of the tube. The bristle portion 2 of Fig. 6 may comprise a first set of bristles 4 configured to extend proximally and a second set of bristles 4 configured to extend distally, the distal direction opposite to the proximal direction. In some embodiments, the bristles 4 extend in a single longitudinal direction in the deployed configuration (either distally or proximally, but not both). That is, in some embodiments, the bristle portion of Fig. 6 may comprise only bristles 4 extending distally, or only bristles 4 extending proximally. The first and second sets of bristles 4 may each be grouped into a single segment located away from the other sets of bristles 4, as illustrated in Fig. 1A (i.e. there may be provided a gap between the bristle segments which is larger than the gap between neighbouring bristles within a single segment). With additional reference Figs. 1A and 1B, the venous ablation device 1 may comprise more than two bristle segments, wherein each segment comprises distally or proximally extending bristles. The core 3 is at least partially integral with the delivery portion 5 of the venous ablation device 1. For example, the delivery portion 5 may comprise one or more of the tube wall 211, the inner mandrel 414 and the filler material which are configured to extend from a proximal end of a delivery catheter to a distal end of the delivery catheter.

As shown in Fig. 6, the plurality of flexible bristles 4 each penetrate through the tube wall 211 such that a portion of each of the flexible bristles 4 is disposed within the lumen of the tube.

The tube wall 211 may have a plurality of holes therein (not shown) through which, for example, a single one or some of the plurality of flexible bristles 4 passes therethrough. The holes may each have substantially the same diameter as the flexible bristle 4 which passes therethrough.

As can be seen from Figure 6, each of the plurality of flexible bristles 4 penetrates through the tube wall 211. A free portion 421a of each of the plurality of flexible bristles 4 extends radially outwardly from the tube, and, in particular, the tube wall 211.

A clamped portion 421b of each of the plurality of flexible bristles 4 is clamped between an inner mandrel 414 disposed within the lumen of the tube and the tube wall 211.

The clamped portions 421b of each of the flexible bristles 4 may be disposed within an annulus 415 defined between the inner mandrel 414 and the tube wall 211.

The inner mandrel 414 may comprise or consist of, for example, any suitable polymer metal or resin.

The outer surface of the inner mandrel 414 and the inner surface of the tube wall 211 provide a clamping force on each of the clamped portions 421b such that the flexible bristles 4 are secured to the core 3.

The portions of the tube wall 211 between some or all of the clamped portions 421b may be configured such that the outer surface of the inner mandrel 414 and the inner surface of the tube wall 211 contact each other (not shown in Figure 6).

Optionally, the portions of the tube wall 211 between some or all of the clamped portions 421b may be configured such that the outer surface of the inner mandrel 414 and the inner surface of the tube wall 211 contact each other such that the core 3 defines a number of distinct, isolated annuluses disposed along the length of the tube. The annuluses of the core 3 may be substantially filled by the clamped portions 421b.

The annulus 415 or annuluses of the core 3 may be filled with any filler material disclosed herein, such as the filler material 212 of Figure 4.

The core 3 and flexible bristles 4 of the venous ablation device 1 may be manufactured by creating, for example, by machining, a plurality of holes in the tube wall 211 of the tube. One of the plurality of flexible bristles 4 may be inserted through each of the plurality of holes in the tube wall 211 such that a portion 421b of each of the flexible bristles 4 extends into the lumen of the tube.

An inner mandrel 414 may be inserted into the lumen of the tube such that the portions 421b are clamped between the inner mandrel 414 and the tube wall 211. Optionally, the tube wall 211 of the tube may be shrunk or mechanically compressed and/or the inner mandrel 414 may be radially expanded once it has been inserted into the lumen of the tube.

One or more of the bristles of Fig. 6 may comprise a thick portion 213 as previously disclosed with reference to Fig. 4.

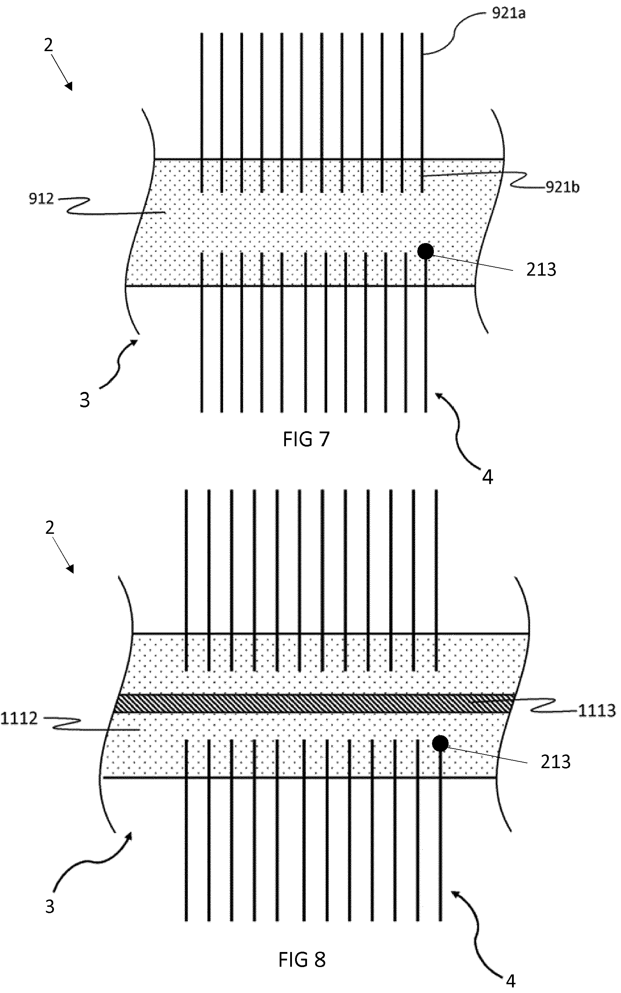

Fig. 7 shows a schematic cross-sectional view of a bristle portion 2 of an NTNT venous ablation device 1 in an unconstrained configuration according to one or more embodiments. For example, the bristle portion 2 shown in Fig. 7 may be provided in the venous ablation device 1 described with reference to Figs. 1A and 1B. In the embodiment shown in Fig. 7, the core 3 of the bristle portion 2 comprises a volume of material 912. The plurality of bristles 4 are each partially disposed within the volume of material 912 such that the material 912 surrounds and secures the plurality of bristles 4. The plurality of bristles 4 extend at least radially outwardly from the core 3. The bristle portion 2 of Fig. 7 may comprise a first set of bristles 4 configured to extend proximally and a second set of bristles 4 configured to extend distally, the distal direction opposite to the proximal direction. In some embodiments, the bristles 4 extend in a single longitudinal direction in the deployed configuration (either distally or proximally, but not both). That is, in some embodiments, the bristle portion of Fig. 7 may comprise only bristles 4 extending distally, or only bristles 4 extending proximally. The first and second sets of bristles 4 may each be grouped into a single segment located away from the other sets of bristles 4, as illustrated in Fig. 1A (i.e. there may be provided a gap between the bristle segments which is larger than the gap between neighbouring bristles within a single segment). Referring additionally to Figs. 1A and 1B, the venous ablation device 1 may comprise more than two bristle segments, wherein each segment comprises distally or proximally extending bristles. The core 3 is at least partially integral with the delivery portion 5 of the venous ablation device 1. For example, the delivery portion 5 may comprise the material 912 which is configured to extend from a proximal end of a delivery catheter to a distal end of the delivery catheter.

In the embodiment shown in Fig. 7, a free portion 921a of each of the plurality of flexible bristles 4 extends radially outwardly from the core 3.

The volume of material 912 may comprise or consist of any of the materials listed for the filler materials described herein.

The core 3 is formed from a material 912. A portion 921b of each of the flexible bristles 4 is disposed within a volume of the material 912 of the core 3. The material 912 surrounds the portion 921b and secures the portion 921b to the core 3.

As shown in Figure 7, the material 912 substantially entirely surrounds and contacts an end portion 921b of each of the flexible bristles 4. Specifically, the material 912 may contact substantially an entire transversely extending edge surface of each of the flexible bristles 4. Referring to Figure 7, the volume of the material 912 is a bulk or homogenous volume of the core 3 formed from a continuous volume of the material 912.

The material 912 of the core 3 engages the portions 921b of the flexible bristles 4. The material 912 acts to secure the plurality of flexible bristles 4 to the core 3.

The material 912 may be an adhesive. In such embodiments, the material 912 may engage the portions 921b of the flexible bristles 4 by adhering or bonding to the portions 921b.

The material 912 may be a curable material or settable material, which is curable or settable upon heating, solvent flashing and/or irradiating. Upon curing or setting, the material 912 may harden so as to secure the flexible bristles 4 to the core 3.

The material 912 may engage the portions 921b of the flexible bristles 4 to mechanically anchor the flexible bristles 4 to the core 3.

As one (or a sub-set) of the plurality of flexible bristles 4 passes into the material 912 of the core 3, the flexible bristles 4 are attached to the core 3 individually or in a (small) sub-set. Accordingly, if the integrity of the attachment of one of the flexible bristles 4 becomes compromised, the integrity of the attachments of the other flexible bristles 4 may not be compromised. For example, if one of the plurality of flexible bristles 4 is dislodged from the core 3, the attachment of the remaining flexible bristles 4 may remain uncompromised, which is not the case when the majority of the attachment force for a particular bristle 4 is provided for by surrounding/neighbouring bristles 4.

Furthermore, the material 912 may be chosen so as to influence the structural properties of the core 3, for example, flexibility.

The core 3 and flexible bristles 4 of the venous ablation device may be manufactured by providing a core 3 formed from a material 912 and providing a plurality of flexible bristles 4 such that a portion 921b of each of the flexible bristles 4 is disposed within a volume of the material 912.

For example, the portions 921b of the flexible bristles 4 may be inserted into the material 912. Thereafter, the material 912 may be cured or set such that the material 912 surrounds the portions 921b and secures the portions 921b to the core 3, for example, by the hardening and/or contraction of the material 912.

In certain embodiments, the core 3 may be formed by molding the material 912. For example, the portions 921b of the flexible bristles 4 may be inserted into a mold cavity defined by a mold. A material to be molded may be inserted into the mold cavity. The material may be allowed to set such that the material 912 surrounds and secures the flexible bristles 4. The mold may then be removed to leave the core 3 and flexible bristles 4 secured thereto.

In certain embodiments, a mold is not required. For example, the material 912 may be shaped into the core 3. Thereafter, the plurality of flexible bristles 4 may be inserted into the shaped material 912 such that the portions 921b are disposed within a volume of the material 912. Thereafter, the material 912 may optionally be cured or set such that the material 912 surrounds the portions 921b and secures the portions 921b to the core 3, for example, by the hardening and/or contraction of the material 912.

One or more of the bristles of Fig. 7 may comprise a thick portion 213 as previously disclosed with reference to Fig. 4.

Fig. 8 shows a schematic cross-sectional view of a bristle portion 2 of an NTNT venous ablation device 1 in an unconstrained configuration according to one or more embodiments. For example, the bristle portion 2 shown in Fig. 8 may be provided in the venous ablation device 1 described with reference to Figs. 1A and 1B. In the embodiment shown in Fig. 8, the core 3 of the bristle portion 2 comprises a volume of material 1112. The plurality of bristles 4 are each partially disposed within the volume of material 1112 such that the material 1112 surrounds and secures the plurality of bristles 4. The plurality of bristles 4 extend at least radially outwardly from the core 3. The bristle portion 2 of Fig. 8 may comprise a first set of bristles 4 configured to extend proximally and a second set of bristles 4 configured to extend distally, the distal direction opposite to the proximal direction. In some embodiments, the bristles 4 extend in a single longitudinal direction in the deployed configuration (either distally or proximally, but not both). That is, in some embodiments, the bristle portion 2 of Fig. 8 may comprise only bristles 4 extending distally, or only bristles 4 extending proximally. The first and second sets of bristles 4 may each be grouped into a single segment located away from the other sets of bristles 4, as illustrated in Fig. 1A (i.e. there may be provided a gap between the bristle segments which is larger than the gap between neighbouring bristles within a single segment). Referring additionally to Figs. 1A and 1B, the venous ablation device 1 may comprise more than two bristle segments, wherein each segment comprises distally or proximally extending bristles 4. The core 3 is at least partially integral with the delivery portion 5 of the venous ablation device 1. For example, the delivery portion 5 may comprise one or more of the material 1112 and structural member 1113 which are configured to extend from a proximal end of a delivery catheter to a distal end of the delivery catheter.

The embodiment shown in Fig. 8 is similar to the embodiment of Fig. 7 in that the venous ablation device has a core 3 formed from a material 1112, and where a portion of each of the flexible bristles 4 is disposed within a volume of the material 1112 and a free portion extends radially outwardly. The volume of material 1112 may comprise or consist of any of the materials listed for the filler materials described herein.

However, in the embodiment shown in Fig. 8, the core 3 further comprises a structural member 1113. The structural member 1113 may be disposed at least partially or entirely within the material 1112 of the core 3. The structural member 1113 may have a lower flexibility than the material 1112 which provides structural support to the core 3.

The structural member 1113 may extend longitudinally along the length of the core 3. The structural member 1113 may be elongated or rod-shaped.

The structural member 1113 may be disposed within the material 1112 before the material 1112 is cured or set so as to secure the structural member 1113 within the material 1112 of the core. Alternatively, the structural member 1113 may be disposed within the material 1112 by inserting it into the material 112 after it has been cured or set, for example, by boring a hole within the material 1112 using the structural member 1113 itself and/or another device, such as a drill.

It is noted that in Figs. 4 to 8, an end of the flexible bristles 4 terminates within the core 3. However, some or all of the flexible bristles 4 of the embodiments described with reference to Figs. 4 to 8 may extend through the core 3 such that both ends of the flexible bristles are exterior to the core 3. The means and methods described in relation to Figs. 4 to 8 are equally applicable to such embodiments.

For example, Fig. 4 shows a bristle 4a penetrating through the tube wall 211 at two locations (i.e. a first location and a second location). Both of the ends of the flexible bristle 4a are disposed outside the core 3. Both ends extend radially outwardly from the core 3, and, in particular, the tube wall 211.

The above-noted first location and second location may be chosen in various manners.

In this regard, reference is made to bristle 4a which shows the first and second locations on substantially opposite sides of the circumference of the tube.

In other embodiments, the first and second locations may be, for example, in the same quarter of the circumference of the core 3. In other embodiments, the first and second locations are in the same half, third, fifth or sixth of the circumference of the core 3. For example, the first and second locations for bristle 4b are not diametrically opposite one another so that the bristle 4b is curved within the core 3.

The venous ablation devices disclosed herein may generally be manufactured by the following process:
providing a core and a delivery portion configured to deliver the bristle portion through a delivery catheter, the delivery portion at least partly integral with the bristle portion;
providing a plurality of resiliently flexible bristles to the core so that the plurality of bristles penetrate through the core; and
securing the bristles to the core so that the plurality of bristles extend at least radially outwardly from the core the bristles having a collapsed delivery configuration and an expanded deployed configuration in which the bristles are configured to contact and denude the vessel wall.

In any of the embodiments described with reference to Fig. 2A, the venous ablation device may be manufactured as described with reference to Figs. 3A and 3B.

In any of the embodiments wherein the core comprises a tube having a tube wall, the steps of providing and securing the bristles to the core may comprises penetrating the plurality of flexible bristles through a plurality of holes in the tube wall (e.g. tube wall 211). The plurality of flexible bristles may be secured to the tube wall by one or more of: mechanically compressing the tube; melting a portion of the tube when the bristles penetrate the tube wall; and shrinking the tube wall chemically or by heating the tube wall.

In any of the embodiments described wherein the core comprises a tube having a tube wall, the step of securing the bristles to the core may comprise clamping the plurality of flexible bristles between opposing sides of the tube wall (e.g. tube wall 211).

In any of the embodiments wherein the core comprises a tube comprising a tube wall, the step of securing the bristles to the core may comprise clamping the plurality of flexible bristles between an inner surface of the tubular wall (e.g. tube wall 211) and an outer surface of a mandrel (e.g. mandrel 414) disposed in a lumen of the tubular wall.

In any of the embodiments described wherein the core comprises a tube comprising tube wall, the method of manufacture may comprise providing a filler material (e.g. filler material 212) within a lumen of the tube to secure the bristles in the tube. The method may comprise curing or setting the filler material.

In any of the embodiments described wherein the core comprises a volume of material (e.g. material 912 or 1112), the steps of providing and securing the bristles to the core may comprise partially disposing each of the plurality of bristles within the volume of material such that the material surrounds and secures the plurality of bristles. Optionally the method further may further comprise providing a structural member disposed at least partially within the volume of material. The bristles may be secured to the volume of material by one or more of: adhering or bonding the material to the plurality of bristles; mechanically anchoring the bristles to the material; and curing or setting the material.

Any of the venous ablation devices disclosed herein may be used in a method of closing a blood vessel of a patient. In the method, a delivery catheter may be inserted into the vasculature of the patient. A distal portion of the delivery catheter may be positioned at a target site in the blood vessel using any suitable technique. For example, a guidewire may be navigated to the target site in the blood vessel and the delivery catheter may be translated over the guidewire to reach the blood vessel. A venous ablation device according to any of the embodiments disclosed herein may be delivered to the distal portion of the delivery catheter, for example by pushing the delivery portion through the delivery catheter, and deployed from the delivery catheter so that the flexible bristles contact the vessel wall (for example as shown in Fig. 1B). The venous ablation device may be deployed by pinning the venous ablation device and moving the delivery catheter proximally, or by pinning the delivery catheter and moving the venous ablation device distally. The venous ablation device may be translated to denude the vessel wall (for example by translating the venous ablation distally and proximally one or multiple times as indicated by the arrow D in Fig. 1B). The venous ablation device and delivery catheter may subsequently be removed from the patient. The venous ablation device may be repositioned inside the delivery catheter before the venous ablation device and delivery catheter are removed.

In the methods disclosed herein, the order of presentation of the method steps do not denote a chronological order unless a subsequent step requires a previous step to be performed in order to enact the subsequent step.

All of the above are fully within the scope of the present disclosure, and are considered to form the basis for alternative embodiments in which one or more combinations of the above described features are applied, without limitation to the specific combinations disclosed above.

In light of this, there will be many alternatives which implement the teaching of the present disclosure. It is expected that one skilled in the art will be able to modify and adapt the above disclosure to suit its own circumstances and requirements within the scope of the present disclosure, while retaining some or all technical effects of the same, either disclosed or derivable from the above, in light of his common general knowledge in this art. The invention is defined by the following claims.

## Claims

1. A system including a delivery catheter (7) and a venous ablation device (1) for denuding a vessel wall (9), comprising:
a bristle portion (2), the bristle portion (2) comprising a core (3) and a plurality of resiliently flexible bristles (4) extending at least radially outwardly from the core (3), the bristles (4) having a collapsed delivery configuration, in which the bristles (4) fit inside the delivery catheter (7), and an expanded deployed configuration in which the bristles (4) are configured to contact and denude the vessel wall (9); and
a delivery portion (5) configured to deliver the bristle portion (2) through the delivery catheter (7), the delivery portion (5) at least partially integral with the core (3);
**characterised in that**
the plurality of bristles (4) penetrate through the core (3) such that the core (3) secures the bristles (4);
wherein the plurality of bristles (4) comprise a first set (6a) of bristles and a second set (6b) of bristles, the first set (6a) of bristles configured to extend in a proximal direction in the expanded deployed configuration and the second set (6b) of bristles configured to extend in a distal direction opposite to the proximal direction in the expanded deployed configuration.

2. The system of claim 1, wherein the core (3) comprises a twisted wire portion (3a), the plurality of bristles (4) clamped by the twisted wire portion (3a).

3. The system of claim 1, wherein the core (3) comprises a tubular wall (211), and the plurality of bristles (4) each penetrate the tubular wall (211) so that the tubular wall (211) secures the plurality of bristles (4) to the core (3), optionally wherein the core (3) further comprises a filler material (212) disposed within a lumen (313, 415) defined by the tubular wall (211), the filler material (212) surrounding and securing portions of the flexible bristles (4) which are disposed within the lumen (313, 415).

4. The system of claim 3, wherein the plurality of bristles (4) are clamped between two opposing sides of the tubular wall (211), or wherein the core (3) further comprises a mandrel (414) disposed in a lumen (313, 415) of the tubular wall (211), and each of the plurality of bristles (4) are clamped between an outer surface of the mandrel (414) and an inner surface of the tubular wall (211).

5. The system of claim 1, wherein the core (3) comprises a volume of material (912, 1112) and the plurality of bristles (4) are each partially disposed within the volume of material (912, 1112) such that the material surrounds and secures the plurality of bristles (4).

6. The system of claim 5, wherein the core (3) comprises a structural member (1113) disposed at least partially within the volume of material (912, 1112), the structural member (1113) having a lower flexibility than the material.

7. The system of any preceding claim, wherein each of the plurality of bristles (4) comprises a thick portion (213), the thick portion (213) being thicker than a portion of the bristle (4) extending through the core (3).

8. The system of any preceding claim, wherein at least some of the plurality of bristles (4) comprise barbs or hooks (40, 41) disposed along the length of the bristles (4) and/or at the tips of the bristles (4).

9. The system of any preceding claim, further comprising one or more radiopaque markers (8).

10. A method of manufacturing a system for denuding a vessel wall (9), the system comprising a delivery catheter (7) and a venous ablation device (1), the method comprising:
providing a core (3) and a delivery portion (5) configured to deliver a bristle portion (2) through the delivery catheter (7), the delivery portion (5) at least partly integral with the bristle portion (2);
providing a plurality of resiliently flexible bristles (4) to the core (3) so that the plurality of bristles (4) penetrate through the core (3); and
securing the bristles (4) to the core (3) so that the plurality of bristles (4) extend at least radially outwardly from the core (3) the bristles (4) having a collapsed delivery configuration, in which the bristles (4) fit inside the delivery catheter (7), and an expanded deployed configuration in which the bristles (4) are configured to contact and denude the vessel wall (9);
wherein the plurality of bristles (4) comprise a first set (6a) of bristles and a second set (6b) of bristles, the first set (6a) of bristles configured to extend in a proximal direction in the expanded deployed configuration and the second set (6b) of bristles configured to extend in a distal direction opposite to the proximal direction in the expanded deployed configuration.

11. The method of claim 10, wherein the core (3) is a twisted wire portion (3a) and the steps of providing and securing the bristles (4) to the core (3) comprise:
placing the plurality of flexible bristles (4) between two wire elements; and
twisting the two wire elements to form the twisted wire portion (3a), the plurality of bristles (4) clamped between the twisted wire portion (3a).

12. The method of claim 10, wherein the core (3) comprises a tube having a tubular wall (211) and the steps of providing and securing the bristles (4) to the core (3) comprises:
penetrating the plurality of flexible bristles (4) through a plurality of holes in the tubular wall (211), optionally wherein the step of securing the bristles (4) to the core (3) comprises:
clamping the plurality of flexible bristles (4) between opposing sides of the tubular wall (211); or
clamping the plurality of flexible bristles (4) between an inner surface of the tubular wall (211) and an outer surface of a mandrel (414) disposed in a lumen (313, 415) of the tubular wall (211).

13. The method of claim 12, wherein the plurality of flexible bristles (4) are secured to the tube wall (211) by one or more of:
mechanically compressing the tube;
melting a portion of the tube when the bristles (4) penetrate the tube wall (211);
shrinking the tube wall (211) chemically or by heating the tube wall (211).

14. The method of any of claims 12 to 13, further comprising providing a filler material (212) within a lumen (313, 415) of the tube to secure the bristles (4) in the tube.

15. The method of claim 10, wherein the core (3) comprises a volume of material (912, 1112) and the steps of providing and securing the bristles (4) to the core (3) comprises partially disposing each of the plurality of bristles (4) within the volume of material (912, 1112) such that the material surrounds and secures the plurality of bristles (4), optionally wherein the method further comprises providing a structural member (1113) disposed at least partially within the volume of material (912, 1112), optionally wherein the step of securing the bristles (4) to the core (3) comprises one or more of the following:
adhering or bonding the material to the plurality of bristles (4);
mechanically anchoring the bristles (4) to the material;
curing or setting the material.

## Patentansprüche

1. System, einschließlich eines Einführungskatheters (7) und einer Venenablationsvorrichtung (1) zum Denudieren einer Gefäßwand (9), umfassend:
einen Borstenabschnitt (2), wobei der Borstenabschnitt (2) einen Kern (3) und eine Vielzahl von federelastischen Borsten (4) umfasst, die sich zumindest radial nach außen von dem Kern (3) erstrecken, wobei die Borsten (4) eine kollabierte Einführungskonfiguration aufweisen, in der die Borsten (4) in den Einführungskatheter (7) passen, und eine expandierte Einsatzkonfiguration, in der die Borsten (4) ausgebildet sind, die Gefäßwand (9) zu kontaktieren und zu denudieren; und
einen Einführabschnitt (5), der ausgebildet ist, den Borstenabschnitt (2) durch den Einführungskatheter (7) einzuführen, wobei der Einführabschnitt (5) zumindest teilweise einstückig mit dem Kern (3) ist;
**dadurch gekennzeichnet, dass** die Vielzahl von Borsten (4) den Kern (3) durchdringt, sodass der Kern (3) die Borsten (4) sichert;
wobei die Vielzahl von Borsten (4) eine erste Borstengruppe (6a) und eine zweite Borstengruppe (6b) umfasst, wobei die erste Borstengruppe (6a) in der expandierten Einsatzkonfiguration ausgebildet ist, sich in eine proximale Richtung zu erstrecken, und die zweite Borstengruppe (6b) in der expandierten Einsatzkonfiguration ausgebildet ist, sich in eine der proximalen Richtung entgegengesetzte distale Richtung zu erstrecken.

2. System nach Anspruch 1, wobei der Kern (3) einen verdrillten Drahtabschnitt (3a) umfasst, wobei die Vielzahl von Borsten (4) durch den verdrillten Drahtabschnitt (3a) geklemmt ist.

3. System nach Anspruch 1, wobei der Kern (3) eine Rohrwand (211) umfasst und die Vielzahl von Borsten (4) jeweils die Rohrwand (211) durchdringt, sodass die Rohrwand (211) die Vielzahl von Borsten (4) an dem Kern (3) sichert, wahlweise wobei der Kern (3) weiter ein Füllmaterial (212) umfasst, das innerhalb eines durch die Rohrwand (211) definierten Lumens (313, 415) angeordnet ist, wobei das Füllmaterial (212) Abschnitte der flexiblen Borsten (4), die innerhalb des Lumens (313, 415) angeordnet sind, umgibt und sichert.

4. System nach Anspruch 3, wobei die Vielzahl von Borsten (4) zwischen zwei gegenüberliegenden Seiten der Rohrwand (211) geklemmt ist, oder wobei der Kern (3) weiter einen Dorn (414) umfasst, der in einem Lumen (313, 415) der Rohrwand (211) angeordnet ist, und wobei jede der Vielzahl von Borsten (4) zwischen einer Außenfläche des Dorns (414) und einer Innenfläche der Rohrwand (211) geklemmt ist.

5. System nach Anspruch 1, wobei der Kern (3) ein Materialvolumen (912, 1112) umfasst und die Vielzahl von Borsten (4) jeweils teilweise innerhalb des Materialvolumens (912, 1112) angeordnet ist, sodass das Material die Vielzahl von Borsten (4) umgibt und sichert.

6. System nach Anspruch 5, wobei der Kern (3) ein Strukturelement (1113) umfasst, das zumindest teilweise innerhalb des Materialvolumens (912, 1112) angeordnet ist, wobei das Strukturelement (1113) eine geringere Flexibilität als das Material aufweist.

7. System nach einem vorstehenden Anspruch, wobei jede der Vielzahl von Borsten (4) einen dicken Abschnitt (213) umfasst, wobei der dicke Abschnitt (213) dicker ist als ein Abschnitt der Borste (4), der sich durch den Kern (3) erstreckt.

8. System nach einem vorstehenden Anspruch, wobei zumindest einige der Vielzahl von Borsten (4) Widerhaken oder Haken (40, 41) umfassen, die entlang der Länge der Borsten (4) und/oder an den Spitzen der Borsten (4) angeordnet sind.

9. System nach einem vorstehenden Anspruch, weiter umfassend eine oder mehrere röntgendichte Markierungen (8).

10. Verfahren zur Herstellung eines Systems zum Denudieren einer Gefäßwand (9), wobei das System einen Einführungskatheter (7) und eine Venenablationsvorrichtung (1) umfasst, wobei das Verfahren Folgendes umfasst:
Bereitstellen eines Kerns (3) und eines Einführabschnitts (5), der ausgebildet ist, einen Borstenabschnitt (2) durch den Einführungskatheter (7) einzuführen, wobei der Einführabschnitt (5) zumindest teilweise einstückig mit dem Borstenabschnitt (2) ist;
Bereitstellen einer Vielzahl von federelastischen Borsten (4) an dem Kern (3), sodass die Vielzahl von Borsten (4) den Kern (3) durchdringt; und
Sichern der Borsten (4) an dem Kern (3), sodass sich die Vielzahl von Borsten (4) zumindest radial nach außen von dem Kern (3) erstreckt, wobei die Borsten (4) eine kollabierte Einführungskonfiguration aufweisen, in der die Borsten (4) in den Einführungskatheter (7) passen, und eine expandierte Einsatzkonfiguration, in der die Borsten (4) ausgebildet sind, die Gefäßwand (9) zu kontaktieren und zu denudieren;
wobei die Vielzahl von Borsten (4) eine erste Borstengruppe (6a) und eine zweite Borstengruppe (6b) umfasst, wobei die erste Borstengruppe (6a) in der expandierten Einsatzkonfiguration ausgebildet ist, sich in eine proximale Richtung zu erstrecken, und die zweite Borstengruppe (6b) in der expandierten Einsatzkonfiguration ausgebildet ist, sich in eine der proximalen Richtung entgegengesetzte distale Richtung zu erstrecken.

11. Verfahren nach Anspruch 10, wobei der Kern (3) ein verdrillter Drahtabschnitt (3a) ist und die Schritte des Bereitstellens und Sicherns der Borsten (4) an dem Kern (3) Folgendes umfassen:
Platzieren der Vielzahl von flexiblen Borsten (4) zwischen zwei Drahtelementen; und
Verdrillen der zwei Drahtelemente zur Bildung des verdrillten Drahtabschnitts (3a), wobei die Vielzahl von Borsten (4) zwischen dem verdrillten Drahtabschnitt (3a) geklemmt ist.

12. Verfahren nach Anspruch 10, wobei der Kern (3) ein Rohr mit einer Rohrwand (211) umfasst und die Schritte des Bereitstellens und Sicherns der Borsten (4) an dem Kern (3) Folgendes umfassen:
Durchdringen der Vielzahl von flexiblen Borsten (4) durch eine Vielzahl von Löchern in der Rohrwand (211), wahlweise wobei der Schritt des Sicherns der Borsten (4) an dem Kern (3) Folgendes umfasst:
Klemmen der Vielzahl von flexiblen Borsten (4) zwischen gegenüberliegenden Seiten der Rohrwand (211); oder
Klemmen der Vielzahl von flexiblen Borsten (4) zwischen einer Innenfläche der Rohrwand (211) und einer Außenfläche eines Dorns (414), der in einem Lumen (313, 415) der Rohrwand (211) angeordnet ist.

13. Verfahren nach Anspruch 12, wobei die Vielzahl von flexiblen Borsten (4) an der Rohrwand (211) gesichert wird durch eines oder mehrere von:
mechanisches Komprimieren des Rohrs;
Schmelzen eines Abschnitts des Rohrs, wenn die Borsten (4) die Rohrwand (211) durchdringen;
chemisches Schrumpfen der Rohrwand (211) oder Schrumpfen durch Erhitzen der Rohrwand (211).

14. Verfahren nach einem der Ansprüche 12 bis 13, weiter umfassend das Bereitstellen eines Füllmaterials (212) innerhalb eines Lumens (313, 415) des Rohrs, um die Borsten (4) in dem Rohr zu sichern.

15. Verfahren nach Anspruch 10, wobei der Kern (3) ein Materialvolumen (912, 1112) umfasst und die Schritte des Bereitstellens und Sicherns der Borsten (4) an dem Kern (3) das teilweise Anordnen jeder der Vielzahl von Borsten (4) innerhalb des Materialvolumens (912, 1112) umfassen, sodass das Material die Vielzahl von Borsten (4) umgibt und sichert, wahlweise wobei das Verfahren weiter das Bereitstellen eines Strukturelements (1113) umfasst, das zumindest teilweise innerhalb des Materialvolumens (912, 1112) angeordnet ist, wahlweise wobei der Schritt des Sicherns der Borsten (4) an dem Kern (3) eines oder mehrere der folgenden umfasst:
Anhaften oder Verbinden des Materials mit der Vielzahl von Borsten (4);
mechanisches Verankern der Borsten (4) an dem Material;
Aushärten oder Erstarren des Materials.

## Revendications

1. Système incluant un cathéter de délivrance (7) et un dispositif (1) d'ablation veineuse pour dénuder une paroi de vaisseau (9), comprenant :
une portion à soies (2), la portion à soies (2) comprenant une âme (3) et une pluralité de soies souples et élastiques (4) s'étendant au moins radialement vers l'extérieur à partir de l'âme (3), les soies (4) présentant une configuration repliée, de délivrance, dans laquelle les soies (4) prennent place à l'intérieur du cathéter de délivrance (7), et une configuration déployée, d'extension, dans laquelle les soies (4) sont configurées de manière à venir en contact avec la paroi de vaisseau (9) et à dénuder cette paroi ; et
une portion de délivrance (5), configurée de manière à délivrer la portion à soies (2) à travers le cathéter de délivrance (7), la portion de délivrance (5) étant au moins partiellement d'un seul tenant avec l'âme (3) ;
**caractérisé en ce que** la pluralité de soies (4) traverse l'âme (3) de sorte que l'âme (3) fixe les soies (4) ;
dans lequel la pluralité de soies (4) comprend un premier ensemble (6a) de soies et un deuxième ensemble (6b) de soies, le premier ensemble (6a) de soies étant configuré de manière à s'étendre dans une direction proximale dans la configuration déployée, d'extension, et le deuxième ensemble (6b) de soies étant configuré de manière à s'étendre dans une direction distale opposée à la direction proximale dans la configuration déployée, d'extension.

2. Système selon la revendication 1, dans lequel l'âme (3) comprend une portion de fil torsadé (3a), la pluralité de soies (4) étant pincée par la portion de fil torsadé (3a).

3. Système selon la revendication 1, dans lequel l'âme (3) comprend une paroi tubulaire (211), et les soies de la pluralité de soies (4) traversent chacune la paroi tubulaire (211) de sorte que la paroi tubulaire (211) fixe la pluralité de soies (4) à l'âme (3), dans lequel, facultativement, l'âme (3) comprend en outre un matériau de remplissage (212) placé dans une lumière (313, 415) définie par la paroi tubulaire (211), le matériau de remplissage (212) entourant et fixant des portions des soies souples et élastiques (4) qui sont placées dans la lumière (313, 415).

4. Système selon la revendication 3, dans lequel les soies de la pluralité de soies (4) sont pincées entre deux côtés opposés de la paroi tubulaire (211), ou dans lequel l'âme (3) comprend en outre un mandrin (414) disposé dans une lumière (313, 415) de la paroi tubulaire (211), et chacune des soies (4) de la pluralité est pincée entre une surface externe du mandrin (414) et une surface interne de la paroi tubulaire (211).

5. Système selon la revendication 1, dans lequel l'âme (3) comprend un volume de matériau (912, 1112) et chaque soie de la pluralité de soies (4) est partiellement disposée dans le volume de matériau (912, 1112) de sorte que le matériau entoure et fixe la pluralité de soies (4).

6. Système selon la revendication 5, dans lequel l'âme (3) comprend un élément structurel (1113) disposé au moins partiellement dans le volume de matériau (912, 1112), l'élément structurel (1113) présentant une flexibilité inférieure à celle du matériau.

7. Système selon une quelconque revendication précédente, dans lequel chacune des soies (4) de la pluralité comprend une portion épaisse (213), la portion épaisse (213) étant plus épaisse qu'une portion de la soie (4) s'étendant à travers l'âme (3).

8. Système selon une quelconque revendication précédente, dans lequel au moins certaines des soies (4) de la pluralité comprennent des barbules ou des crochets (40, 41) aménagés le long des soies (4) et/ou au niveau des pointes des soies (4).

9. Système selon une quelconque revendication précédente, comprenant en outre un ou plusieurs marqueurs radio-opaques (8).

10. Procédé de fabrication d'un système pour dénuder une paroi de vaisseau (9), le système comprenant un cathéter de délivrance (7) et un dispositif (1) d'ablation veineuse, le procédé comprenant ce qui suit :
le fait de se mettre à disposition une âme (3) et une portion de délivrance (5) configurées de manière à délivrer une portion à soies (2) à travers le cathéter de délivrance (7), la portion de délivrance (5) étant au moins partiellement d'un seul tenant avec la portion à soies (2) ;
le fait de prévoir une pluralité de soies souples et élastiques (4) sur l'âme (3) de sorte que la pluralité de soies (4) traverse l'âme (3) ; et
le fait de fixer les soies (4) à l'âme (3) de sorte que la pluralité de soies (4) s'étendent au moins radialement vers l'extérieur à partir de l'âme (3), les soies (4) présentant une configuration repliée, de délivrance, dans laquelle les soies (4) prennent place à l'intérieur du cathéter de délivrance (7), et une configuration déployée, d'extension, dans laquelle les soies (4) sont configurées de manière à venir en contact avec la paroi de vaisseau (9) et dénuder cette paroi ;
dans lequel la pluralité de soies (4) comprend un premier ensemble (6a) de soies et un deuxième ensemble (6b) de soies, le premier ensemble (6a) de soies étant configuré de manière à s'étendre dans une direction proximale dans la configuration déployée, d'extension, et le deuxième ensemble (6b) de soies étant configuré de manière à s'étendre dans une direction distale opposée à la direction proximale dans la configuration déployée, d'extension.

11. Procédé selon la revendication 10, dans lequel l'âme (3) est une portion de fil torsadé (3a) et les étapes de fourniture et de fixation des soies (4) à l'âme (3) comprennent ce qui suit :
le fait de placer la pluralité de soies flexibles (4) entre deux éléments de fil ; et
le fait de torsader les deux éléments de fil pour former la portion de fil torsadé (3a), la pluralité de soies (4) étant pincée dans la portion de fil torsadé (3a).

12. Procédé selon la revendication 10, dans lequel l'âme (3) comprend un tube présentant une paroi tubulaire (211) et les étapes de fourniture et de fixation des soies (4) à l'âme (3) comprennent ce qui suit :
le fait d'engager la pluralité de soies flexibles (4) à travers une pluralité de trous présents dans la paroi tubulaire (211), dans lequel, facultativement, l'étape de fixation des soies (4) à l'âme (3) comprend :
le pincement de la pluralité de soies flexibles (4) entre des côtés opposés de la paroi tubulaire (211) ; ou
le pincement de la pluralité de soies flexibles (4) entre une surface interne de la paroi tubulaire (211) et une surface externe d'un mandrin (414) disposé dans une lumière (313, 415) de la paroi tubulaire (211).

13. Procédé selon la revendication 12, dans lequel la pluralité de soies flexibles (4) est fixée à la paroi tubulaire (211) par un ou plusieurs parmi :
une compression mécanique du tube ;
une fusion d'une portion du tube lorsque les soies (4) traversent la paroi tubulaire (211) ;
un rétrécissement de la paroi tubulaire (211) chimiquement ou par chauffage de la paroi tubulaire (211).

14. Procédé selon l'une quelconque des revendications 12 à 13, comprenant en outre le fait de prévoir un matériau de remplissage (212) dans une lumière (313, 415) du tube pour fixer les soies (4) dans le tube.

15. Procédé selon la revendication 10, dans lequel l'âme (3) comprend un volume de matériau (912, 1112), et les étapes de fourniture et de fixation des soies (4) à l'âme (3) comprennent le fait de disposer partiellement chacune des soies (4) de la pluralité dans le volume de matériau (912, 1112) de sorte que le matériau entoure et fixe la pluralité de soies (4), dans lequel, facultativement, le procédé comprend en outre le fait de prévoir un élément structurel (1113) disposé au moins partiellement dans le volume de matériau (912, 1112), dans lequel, facultativement, l'étape de fixation des soies (4) à l'âme (3) comprend un ou plusieurs parmi :
une adhésion ou liaison du matériau à la pluralité de soies (4) ;
un ancrage mécanique des soies (4) au matériau ;
un durcissement ou une prise du matériau.
